# EUROPEAN PATENT SPECIFICATION

(11) **EP 2 659 901 B1**
(45) Date of publication and mention of the grant of the patent: **21.01.2015**
(21) Application number: 13171390.1
(22) Date of filing: 17.04.2009
(51) Int. Cl.: A61K 38/00, C12N 9/10

(54) **Recombinant deamidated gliadin antigen**
Rekombinantes deamidiertes Gliadin-Antigen
Antigène de gliadine recombinante déamidée

(30) Priority: 21.04.2008 US 46693 P
(43) Date of publication of application: 06.11.2013
(62) Divisional of application: 09734340.4
(73) Proprietor: Bio-Rad Laboratories, Inc., Hercules, CA 94547 (US)
(72) Inventor: Watkins, Michael I., Vacaville, CA 95688 (US); Marr, Gregory A., Martinez, CA 94553 (US); Yang, Xiaoyun, Vallejo, CA 94591 (US); Bruehl, Richard, Berkeley, CA 94708 (US); Shan, Daming, N. Shoreline, WA 98133 (US); Coleman, Patrick F., Edmonds, WA 98026 (US)
(74) Representative: Von Kreisler Selting Werner - Partnerschaft von Patentanwälten und Rechtsanwälten mbB

(56) References cited:
- WO-A2-02/083722
- WO-A2-2008/145084
- US-A1- 2004 023 848
- US-A1- 2006 240 475
- ALEANZI M ET AL: "Celiac disease: antibody recognition against native and selectively deamidated gliadin peptides.", CLINICAL CHEMISTRY NOV 2001 LNKD- PUBMED:11673371, vol. 47, no. 11, 1 November 2001 (2001-11-01), pages 2023-2028, XP002666192, ISSN: 0009-9147

## Description

### CROSS-REFERENCES TO RELATED APPLICATIONS

This application claims the benefit of priority to U.S. Application No. 61/046693, filed April 21, 2008.

### BACKGROUND OF THE INVENTION

Celiac disease (CD) is a severe gastrointestinal disease that has a strong genetic component. CD is characterized by a permanent intolerance of proteins from wheat, barley, rye, and oats. Although the physiopathology of CD is not completely understood it is clear that the presence of the toxic proteins in the patient's diet causes a total or partial damage of intestinal mucosa (Brandtzaeg, P. 1997. Mechanisms of gastrointestinal reactions to food. Environmental Toxicology and Pharmacology 4;9-24) leading to severe malabsorption syndromes and causing diarrhea, vomiting, abdominal pain, anorexia, retarded growth, malnutrition and anemia. CD has been associated with a higher risk for intestinal cancer in non-diagnosed and untreated patients (Holmes GKT, 1989. Malignancy in coeliac disease-effect of a gluten-free diet, Gut 30;333-338). CD affects mainly children under three years old, but it is also common in adults, and sometimes is clinically atypical or asymptomatic (Ferguson A, et al. 1992. Definitions and diagnostic criteria of latent and potential coeliac disease. Ed by Aurricchio S, Visakorpi J K, in Epidemiology of CD. Dyn Nutr Res, Basel, Karger 2;119-127). CD is more frequent in patients with other genetic or autoimmune disease, as insulin dependent diabetes mellitus, Down syndrome, selective IgA deficiency, and dermatitis herpetiformis (Sirgus N et al. 1993. Prevalence of coeliac disease in diabetic children and adolescents in Sweden. Acta Pediatr 66;491-494; Zubillaga P et al. 1993. Down syndrome and coeliac disease. J Pediatr Gastroenterol Nutr 16:168-171; Boyce N 1997).

The clinical symptoms of CD could be confused with those produced by other gastrointestinal diseases. In these cases CD is misdiagnosed and patients do not receive the specific treatment, that is, a complete elimination of gluten in their diet. On the other hand, if a non-celiac patient is wrongly diagnosed as celiac, he would undergo an unnecessary gluten free diet for his whole life. Accordingly, a precise diagnosis of CD is essential. Currently the standard for CD diagnosis is intestinal biopsy, repeated three times: at the onset of the clinical symptoms, after several months on a gluten free diet, and after a challenge with gluten.

Because intestinal biopsy is an invasive method and precise serological tests have been developed, the above criteria have been revised (Walker-Smith et al. 1990. Revised criteria for diagnosis of coeliac disease. Report of Working group of European Society of Pediatric Gastroenterology and Nutrition. Arch Dis Child 65:909-911). Currently, serological tests can be done at the onset of clinical symptoms and when they are positive, a confirmatory intestinal biopsy will be indicated. The response to the treatment with a gluten-free diet can be also followed by serological tests. If discrepancies occur between the clinical response to the treatment and the result of serological tests a second intestinal biopsy would be indicated. Several serological tests have been developed for celiac disease diagnosis, as the detection of antibodies to cellular antigens, or antibodies to food antigens, like gliadins. There are diagnostic kits for the detection of: Anti-endomysial antibodies, Anti-reticulin antibodies, Anti-gliadin antibodies, and Anti-tissue Transglutaminase antibodies.

Anti-gliadin antibodies (AGA) have been extensively used for serological diagnosis of CD (Stem M et al. 1996. Validation and standardization of serological screening tests for coeliac disease in 1996. 3 rd EMRC/ESPGAN Workshop, Dec 5-8, 1996, Molsheim, France, pp:9-24; Catassi C et al. 1999. Quantitative antigliadin antibody measurement in clinical practice: an Italian multicenter study. Ital J Gastroenterol Hapatol 31; 366-370). AGA are mainly detected by ELISA (Enzyme-Linked Immunosorbent Assay), a simpler, more objective method than IFA (indirect immunofluorescent antibody analysis), and can be used for the analysis of a large number of samples. Nevertheless AGA are less specific for CD than endomysal antibodies (EMA) and the detection of antibodies to either IgA or IgG isotypes requires two independent assays. Recently a visual immunoassay for the detection of AGA, which solves some of these problems, has been reported (Garrote J A, Sorell L, Alfonso P et al 1999. A simple visual immunoassay for the screening of coeliac disease. Eur. J Clin Invest 29; 697-699; Spanish Office for Patents and Marks No. 9801067).

In 1997, Dietrich *et al.* identified tissue transglutaminase (tTG), an 85 kDa protein, as the major auto antigen detected by anti-endomysial antibodies (Dietrich W et al. 1997. Identification of tissue transglutaminase as the auto antigen of celiac disease. Nat Med. 3:797-801). Detection of anti-tTG antibodies had been reported lately in ELISA or radioligand (RLA) formats based on tTG from guinea pig liver extracts or recombinant human tTG cloned from different tissues (Sulkanen S et al. 1998. Tissue transglutaminase autoantibody enzyme-linked immunosorbent assay in detecting celiac disease. Gastroenterology 115:1322-1328; Siessler J et al. 1999. Antibodies to human recombinant tissue transglutaminase measured by radioligand assay: Evidence for high diagnostic sensitivity for celiac disease. Horm Metab Res 31; 375-379).

Prior art methods for detection of celiac disease use specific gliadin epitopes or pieces of the gliadin protein in an assay, that lead to both false-negatives and false-positives. What is needed is an assay that provides new antigens containing a more inclusive set of epitopes that provides a more accurate assay for celiac disease. Surprisingly, the present invention meets this and other needs.

### SUMMARY OF THE INVENTION

In one aspect, the present invention provides a method for determining whether a subject is suffering from celiac disease by contacting a sample of bodily fluid from the subject, with an antigen formed from a gliadin fusion protein immobilized on a solid support. The gliadin fusion protein of the antigen includes a recombinant deamidated gliadin linked to a tag such as a Glutathione-S transferase (GST) protein. The antigen is prepared by immobilizing onto the solid support the gliadin fusion protein via the tag. The antigen can further include tissue Transglutaminase (tTG) cross-linked to the gliadin fusion protein. When tTG is present, the tTG and recombinant deamidated gliadin are mixed together prior to immobilization on the solid phase.

Current state of the art methods for detecting celiac disease utilize recombinant and natural gliadin, gliadin peptides, deamidated gliadin peptides or tissue transglutaminase as antigens for the detection of the corresponding antibodies. It is suggested that deamidated gliadin, tTG and a complex of deamidated gliadin and tTG are the disease state antigens that are presented by T-cells for the generation of antibodies. It is known that the presence of antibodies to natural gliadin is not disease specific, as evidenced by the presence of high prevalence of anti-gliadin IgG antibodies in healthy patients. Gliadin is not a homogenous protein but rather a class of proteins whose sequences vary by species (e.g. wheat, rye and barley) and strain and even within a strain. As a result, current assays either do not possess a complete epitope repertoire (e.g. synthetic or recombinant deamidated gliadin peptides) or generate false positive results when the non-deamidated antigen is used. The present invention addresses the deficiencies of the prior art methods by combining a recombinant deamidated gliadin protein with a tag immobilized on a solid support.

In another aspect, the present invention provides an antigen for detecting celiac disease, where the antigen includes a recombinant deamidated gliadin covalently linked to a tag, forming a gliadin fusion protein. The tag is immobilized on a solid support.

In a third aspect, the present invention provides an antigen for detecting celiac disease prepared by the process of contacting a solid support with a gliadin fusion protein, wherein the gliadin fusion protein includes a recombinant deamidated gliadin covalently linked to a tag, such that the gliadin fusion protein is immobilized on the solid support via the tag. In this manner, the antigen for detecting celiac disease is prepared.

In a fourth aspect, the present invention provides a method for determining whether a subject is suffering from celiac disease, by contacting a sample of bodily fluid from the subject with the antigen described above; and detecting any antibody that has become specifically bound to the antigen, as an indication of the presence of celiac disease in the subject.

In a fifth aspect, the present invention provides a kit including an antigen as described above, a detection reagent, and optionally at least one of buffers, salts, stabilizers and instructions.

In a sixth aspect, the present invention provides an isolated nucleic acid of SEQ ID NO:5.

### BRIEF DESCRIPTION OF THE DRAWINGS

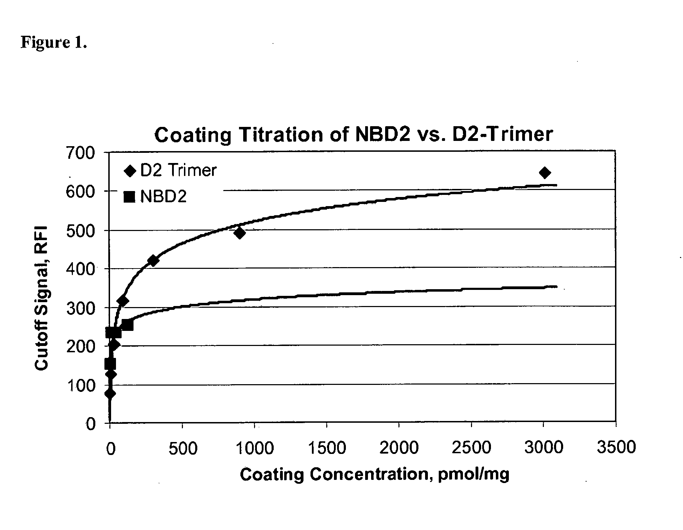

**Figure 1** shows the recombinant deamidated gliadin D2 trimer of the present invention having improved signal-to-noise ratio as compared to the recombinant deamidated gliadin D2 monomer (NBD2).

### DETAILED DESCRIPTION OF THE INVENTION

### I. Definitions

As used herein, the term "contacting" refers to the process of bringing into contact at least two distinct species such that they can react. The resulting reaction product is either produced directly from a reaction between the added reagents or from an intermediate from one or more of the added reagents which can be produced in the reaction mixture.

As used herein, the term "bodily fluid" refers to fluids of a mammal including, but not limited to, aqueous humour, bile, blood and blood plasma, breast milk, interstitial fluid, lymph, mucus, pleural fluid, pus, saliva, serum, sweat, tears, urine, cerebrospinal fluid, synovial fluid or intracellular fluid. One of skill in the art will appreciate that other bodily fluids are useful in the present invention.

As used herein, the term "cross-linker" refers to a bifunctional or multi-functional chemical or biological moiety that is capable of linking two separate moieties together. Examples of cross-linkers useful in the present invention are described below.

As used herein, "antibody" includes reference to an immunoglobulin molecule immunologically reactive with a particular antigen, and includes both polyclonal and monoclonal antibodies. The term also includes genetically engineered forms such as chimeric antibodies (*e.g*., humanized murine antibodies) and heteroconjugate antibodies *(e.g.,* bispecific antibodies). The term "antibody" also includes antigen binding forms of antibodies, including fragments with antigen-binding capability (*e.g*., Fab', F(ab')₂, Fab, Fv and rIgG. *See also,* Pierce Catalog and Handbook, 1994-1995 (Pierce Chemical Co., Rockford, IL). *See also, e.g.,* Kuby, J., Immunology, 3rd Ed., W.H. Freeman & Co., New York (1998). The term also refers to recombinant single chain Fv fragments (scFv). The term antibody also includes bivalent or bispecific molecules, diabodies, triabodies, and tetrabodies. Bivalent and bispecific molecules are described in, *e.g*., Kostelny et al.. (1992) J Immunol 148: 1547, Pack and Pluckthun (1992) Biochemistry 31:1579, Hollinger *et al.,* 1993, *supra,* Gruber et al. (1994) J Immunol :5368, Zhu et al. (1997) Protein Sci 6:781, Hu et al. (1996) Cancer Res. 56:3055, Adams et al. (1993) Cancer Res. 53:4026, and McCartney, et al. (1995) Protein Eng. 8:301.

An antibody immunologically reactive with a particular antigen can be generated by recombinant methods such as selection of libraries of recombinant antibodies in phage or similar vectors, *see, e.g.,* Huse et al., Science 246:1275-1281 (1989); Ward et al., Nature 341:544-546 (1989); and Vaughan et al., Nature Biotech. 14:309-314 (1996), or by immunizing an animal with the antigen or with DNA encoding the antigen.

Typically, an immunoglobulin has a heavy and light chain. Each heavy and light chain contains a constant region and a variable region, (the regions are also known as "domains"). Light and heavy chain variable regions contain four "framework" regions interrupted by three hypervariable regions, also called "complementarity-determining regions" or "CDRs". The extent of the framework regions and CDRs have been defined. The sequences of the framework regions of different light or heavy chains are relatively conserved within a species. The framework region of an antibody, that is the combined framework regions of the constituent light and heavy chains, serves to position and align the CDRs in three dimensional space.

The CDRs are primarily responsible for binding to an epitope of an antigen. The CDRs of each chain are typically referred to as CDR1, CDR2, and CDR3, numbered sequentially starting from the N-terminus, and are also typically identified by the chain in which the particular CDR is located. Thus, a V_{H} CDR3 is located in the variable domain of the heavy chain of the antibody in which it is found, whereas a V_{L} CDR1 is the CDR1 from the variable domain of the light chain of the antibody in which it is found.

References to "V_{H}" or a "VH" refer to the variable region of an immunoglobulin heavy chain of an antibody, including the heavy chain of an Fv, scFv , or Fab. References to "V_{L}" or a "VL" refer to the variable region of an immunoglobulin light chain, including the light chain of an Fv, scFv, dsFv or Fab.

The phrase "single chain Fv" or "scFv" refers to an antibody in which the variable domains of the heavy chain and of the light chain of a traditional two chain antibody have been joined to form one chain. Typically, a linker peptide is inserted between the two chains to allow for proper folding and creation of an active binding site.

A "chimeric antibody" is an immunoglobulin molecule in which (a) the constant region, or a portion thereof, is altered, replaced or exchanged so that the antigen binding site (variable region) is linked to a constant region of a different or altered class, effector function and/or species, or an entirely different molecule which confers new properties to the chimeric antibody, e.g., an enzyme, toxin, hormone, growth factor, drug, etc.; or (b) the variable region, or a portion thereof, is altered, replaced or exchanged with a variable region having a different or altered antigen specificity.

A "humanized antibody" is an immunoglobulin molecule which contains minimal sequence derived from non-human immunoglobulin. Humanized antibodies include human immunoglobulins (recipient antibody) in which residues from a complementary determining region (CDR) of the recipient are replaced by residues from a CDR of a non-human species (donor antibody) such as mouse, rat or rabbit having the desired specificity, affinity and capacity. In some instances, Fv framework residues of the human immunoglobulin are replaced by corresponding non-human residues. Humanized antibodies may also comprise residues which are found neither in the recipient antibody nor in the imported CDR or framework sequences. In general, a humanized antibody will comprise substantially all of at least one, and typically two, variable domains, in which all or substantially all of the CDR regions correspond to those of a non-human immunoglobulin and all or substantially all of the framework (FR) regions are those of a human immunoglobulin consensus sequence. The humanized antibody optimally also will comprise at least a portion of an immunoglobulin constant region (Fc), typically that of a human immunoglobulin (Jones et al., Nature 321:522-525 (1986); Riechmann et al., Nature 332:323-329 (1988); and Presta, Curr. Op. Struct. Biol. 2:593-596 (1992)). Humanization can be essentially performed following the method of Winter and co-workers (Jones et al., Nature 321:522-525 (1986); Riechmann et al., Nature 332:323-327 (1988); Verhoeyen et al., Science 239:1534-1536 (1988)), by substituting rodent CDRs or CDR sequences for the corresponding sequences of a human antibody. Accordingly, such humanized antibodies are chimeric antibodies (U.S. Patent No. 4,816,567), wherein substantially less than an intact human variable domain has been substituted by the corresponding sequence from a non-human species.

"Epitope" or "antigenic determinant" refers to a site on an antigen to which an antibody binds. Epitopes can be formed both from contiguous amino acids or noncontiguous amino acids juxtaposed by tertiary folding of a protein. Epitopes formed from contiguous amino acids are typically retained on exposure to denaturing solvents whereas epitopes formed by tertiary folding are typically lost on treatment with denaturing solvents. An epitope typically includes at least 3, and more usually, at least 5 or 8-10 amino acids in a unique spatial conformation. Methods of determining spatial conformation of epitopes include, for example, x-ray crystallography and 2-dimensional nuclear magnetic resonance. See, e.g., Epitope Mapping Protocols in Methods in Molecular Biology, Vol. 66, Glenn E. Morris, Ed (1996).

As used herein, the term "subject" refers to animals such as mammals, including, but not limited to, primates (*e.g.,* humans), cows, sheep, goats, horses, dogs, cats, rabbits, rats, mice and the like.

As used herein, the term "immobilized" refers to the association of the tTG, the gliadin fusion protein or the tTG-gliadin fusion protein complex with a solid support material through covalent bond formation, ionic bond formation, hydrogen-bonding, dipole-dipole interaction or via Van der Waals interactions. The immobilization can be temporary or permanent.

As used herein, the term "antigen" refers to a molecule that is capable of stimulating an immune response such as by production of antibodies. Antigens of the present invention include solid support immobilized gliadin fusion protein and solid support immobilized tTG-gliadin fusion protein complex. The gliadin fusion protein of the present invention can include both a recombinant deamidated gliadin and a tag, such as Glutathione S-transferase (GST) protein.

As used herein, the term "buffers" refers to any inorganic or organic acid or base that resists changes in pH and maintains the pH around a desired point. Buffering agents useful in the present invention include, but are not limited to, sodium hydroxide, dibasic sodium phosphate anhydrous, and mixtures thereof. One of skill in the art will appreciate that other buffering agents are useful in the present invention.

As used herein, the term "tissue Transglutaminase (tTG)" refers to an enzyme of the transglutaminase family that crosslinks proteins between an amino group of a lysine residue and a carboxamide group of a glutamine residue. This creates an intermolecular or intramolecular bond. tTG can be used to detect celiac disease.

As used herein, the term "gliadin fusion protein" refers to a gliadin protein linked to a tag such as Glutathione S-transferase (GST). The gliadin protein includes a recombinant gliadin protein or a synthetic gliadin protein, among others. Tags are typically other proteins or compounds that can be used as affinity tags for purification, for solubilization, chromatography, as epitope tags, fluorescence tags, and others. Tags useful in the present invention include, but are not limited to, BCCP, c-myc-tag, Calmodulin-tag, FLAG-tag, HA-tag, His-tag, Maltose binding protein-tag, Nus-tag, Glutathione-S-transferase-tag, Green fluorescent protein-tag, Thioredoxin-tag, S-tag, Streptag II, HA-tag, Softag 1, Softag 3, T7-tag, Elastin-like peptides, Chitin-binding domain, and Xylanase 10A. One of skill in the art will appreciate that other proteins are useful in fusion proteins of the present invention.

As used herein, the term "tTG-gliadin fusion protein complex" refers to a complex formed when the tTG and the gliadin fusion protein become linked together. The tTG and the gliadin fusion protein can be linked in a variety of ways, under a variety of reactions. The tTG can be linked to either or both of the tag and the recombinant deamidated gliadin of the gliadin fusion protein.

As used herein, the term "recombinant deamidated gliadin" refers to a deamidated gliadin protein prepared via genetic engineering. Deamidated proteins are those that have had some or all of the free amide functional groups hydrolyzed to carboxylic acids, such as conversion of glutamines to glutamic acid. Recombinant deamidated gliadins useful in the present invention have at least 75% sequence identity to SEQ ID NO:1 or SEQ ID NO:2.

As used herein, the term "crosslinked" refers to the formation of more than one bond between two different chemical moieties. In the present invention, the chemical moieties can be biological species such as proteins, enzymes, antibodies, etc., or solid support materials. The chemical functionality that links the individual chemical moieties that are crosslinked, is termed a "crosslinker". A crosslinker is typically a bifunctional compound that reacts with one reactive functional group on one chemical moiety and one reactive functional group on another chemical moiety, thereby linking the two chemical moieties to each other. The crosslinkers can be homobifunctional crosslinkers or heterobifunctional crosslinkers. Homobifunctional crosslinkers are those where the functional groups of the homobifunctional crosslinker that react with each chemical moiety are the same. Heterobifunctional crosslinkers are those where the functional groups of the heterobifunctional crosslinker that react with each chemical moiety are different. Preferred homobifunctional and heterobifunctional crosslinkers of the present invention are described in greater detail below.

As used herein, the terms "identical" or percent "identity," in the context of two or more nucleic acids or polypeptide sequences, refer to two or more sequences or subsequences that are the same or have a specified percentage of amino acid residues or nucleotides that are the same (*i.e.,* 60% identity, preferably 65%, 70%, 75%, 80%, 85%, 90%, 95%, 96%, 97%, 98% or 99% identity over a specified region), when compared and aligned for maximum correspondence over a comparison window, or designated region as measured using one of the following sequence comparison algorithms or by manual alignment and visual inspection. Such sequences are then said to be "substantially identical." This definition also refers to the compliment of a test sequence.

The phrase "substantially identical," in the context of two nucleic acids or polypeptides, refers to a sequence or subsequence that has at least 40% sequence identity with a reference sequence. Alternatively, percent identity can be any integer from 40% to 100%. More preferred embodiments include at least: 40%, 45%, 50%, 55%, 60%, 65%, 70%, 75%, 80%, 85%, 90%, 95%, 96%, 97%, 98% or 99% compared to a reference sequence using the programs described herein; preferably BLAST using standard parameters, as described below.

For sequence comparison, typically one sequence acts as a reference sequence, to which test sequences are compared. When using a sequence comparison algorithm, test and reference sequences are entered into a computer, subsequence coordinates are designated, if necessary, and sequence algorithm program parameters are designated. Default program parameters can be used, or alternative parameters can be designated. The sequence comparison algorithm then calculates the percent sequence identities for the test sequences relative to the reference sequence, based on the program parameters. For sequence comparison of nucleic acids and proteins, the BLAST and BLAST 2.0 algorithms and the default parameters discussed below are used.

Preferred examples of algorithms that are suitable for determining percent sequence identity and sequence similarity are the BLAST and BLAST 2.0 algorithms, which are described in Altschul et al., Nuc. Acids Res. 25:3389-3402 (1977) and Altschul et al., J. Mol. Biol. 215:403-410 (1990), respectively. BLAST and BLAST 2.0 are used, with the parameters described herein, to determine percent sequence identity for the nucleic acids and proteins of the invention. Software for performing BLAST analyses is publicly available through the National Center for Biotechnology Information (http://www.ncbi.nlm.nih.gov/). This algorithm involves first identifying high scoring sequence pairs (HSPs) by identifying short words of length W in the query sequence, which either match or satisfy some positive-valued threshold score T when aligned with a word of the same length in a database sequence. T is referred to as the neighborhood word score threshold (Altschul *et al., supra*). These initial neighborhood word hits act as seeds for initiating searches to find longer HSPs containing them. The word hits are extended in both directions along each sequence for as far as the cumulative alignment score can be increased. Cumulative scores are calculated using, for nucleotide sequences, the parameters M (reward score for a pair of matching residues; always > 0) and N (penalty score for mismatching residues; always < 0). For amino acid sequences, a scoring matrix is used to calculate the cumulative score. Extension of the word hits in each direction are halted when: the cumulative alignment score falls off by the quantity X from its maximum achieved value; the cumulative score goes to zero or below, due to the accumulation of one or more negative-scoring residue alignments; or the end of either sequence is reached. The BLAST algorithm parameters W, T, and X determine the sensitivity and speed of the alignment. The BLASTN program (for nucleotide sequences) uses as defaults a word length (W) of 11, an expectation (E) of 10, M=5, N=-4 and a comparison of both strands. For amino acid sequences, the BLASTP program uses as defaults a word length of 3, and expectation (E) of 10, and the BLOSUM62 scoring matrix (see Henikoff & Henikoff, Proc. Natl. Acad. Sci. USA 89:10915 (1989)) alignments (B) of 50, expectation (E) of 10, M=5, N=-4, and a comparison of both strands.

The BLAST algorithm also performs a statistical analysis of the similarity between two sequences (see, *e.g.,* Karlin & Altschul, Proc. Nat'l. Acad. Sci. USA 90:5873-5787 (1993)). One measure of similarity provided by the BLAST algorithm is the smallest sum probability (P(N)), which provides an indication of the probability by which a match between two nucleotide or amino acid sequences would occur by chance. For example, a nucleic acid is considered similar to a reference sequence if the smallest sum probability in a comparison of the test nucleic acid to the reference nucleic acid is less than about 0.2, more preferably less than about 0.01, and most preferably less than about 0.001.

An indication that two nucleic acid sequences or polypeptides are substantially identical is that the polypeptide encoded by the first nucleic acid is immunologically cross reactive with the antibodies raised against the polypeptide encoded by the second nucleic acid, as described below. Thus, a polypeptide is typically substantially identical to a second polypeptide, for example, where the two peptides differ only by conservative substitutions. Another indication that two nucleic acid sequences are substantially identical is that the two molecules or their complements hybridize to each other under stringent conditions. Yet another indication that two nucleic acid sequences are substantially identical is that the same primers can be used to amplify the sequence.

As used herein, the terms "nucleic acid" and "polynucleotide" are used synonymously and refer to a single or double-stranded polymer of deoxyribonucleotide or ribonucleotide bases read from the 5' to the 3' end. A nucleic acid of the present invention will generally contain phosphodiester bonds, although in some cases, nucleic acid analogs may be used that may have alternate backbones, comprising, *e.g.,* phosphoramidate, phosphorothioate, phosphorodithioate, or O-methylphosphoroamidite linkages (see Eckstein, Oligonucleotides and Analogues: A Practical Approach, Oxford University Press); and peptide nucleic acid backbones and linkages. Other analog nucleic acids include those with positive backbones; non-ionic backbones, and non-ribose backbones. Thus, nucleic acids or polynucleotides may also include modified nucleotides, that permit correct read through by a polymerase. "Polynucleotide sequence" or "nucleic acid sequence" includes both the sense and antisense strands of a nucleic acid as either individual single strands or in a duplex. As will be appreciated by those in the art, the depiction of a single strand also defines the sequence of the complementary strand; thus the sequences described herein also provide the complement of the sequence. Unless otherwise indicated, a particular nucleic acid sequence also implicitly encompasses variants thereof (*e.g.,* degenerate codon substitutions) and complementary sequences, as well as the sequence explicitly indicated. The nucleic acid may be DNA, both genomic and cDNA, RNA or a hybrid, where the nucleic acid may contain combinations of deoxyribo- and ribo-nucleotides, and combinations of bases, including uracil, adenine, thymine, cytosine, guanine, inosine, xanthine hypoxanthine, isocytosine, isoguanine, etc

As used herein, the phrase "a nucleic acid sequence encoding" refers to a nucleic acid which contains sequence information for a structural RNA such as rRNA, a tRNA, or the primary amino acid sequence of a specific protein or peptide, or a binding site for a transacting regulatory agent. This phrase specifically encompasses degenerate codons *(i.e.,* different codons which encode a single amino acid) of the native sequence or sequences that may be introduced to conform with codon preference in a specific host cell.

As used herein, the term "specifically bound" refers to the capturing or entrapment of the antigen of the present invention by an antibody that is indicative of the presence of celiac disease.

### II. Antigen

The present invention provides an antigen and method for detection of celiac disease. The antigen includes a gliadin fusion protein immobilized on a solid support material. The gliadin fusion protein includes both a recombinant deamidated gliadin and a tag. The antigen can optionally include tissue Transglutaminase (tTG). When present, the gliadin fusion protein and tTG can be covalently linked prior to immobilization on the solid support, such as via transamidation, to form a tTG-gliadin fusion protein complex. Following immobilization of the tTG-gliadin fusion protein complex on the solid support, the gliadin fusion protein and the tTG can be cross-linked using suitable cross-linkers.

In some embodiments, the present invention provides an antigen for detecting celiac disease. The antigen of the present invention includes the solid support bound gliadin fusion protein described below.

### A. Gliadin Fusion Protein

The gliadin fusion protein useful in the present invention includes a recombinant deamidated gliadin and a tag. One of skill in the art will recognize that many recombinant gliadin proteins are useful in the method of the present invention. In some embodiments, the recombinant gliadin protein can include D2 (Aleanzi et al, Clin Chem 2001, 47 (11), 2023), peptide sequence: QPEQPQQSFPEQERPF (SEQ ID NO:1). The recombinant gliadin protein can also include variants of D2, represented by the following formula:

X¹PX²X³PX⁴X⁵SFPX⁶X⁷X⁸RPF

wherein each X is either glutamine (Q) or glutamic acid (E) such that at least one X is glutamine and at least one X is glutamic acid (SEQ ID NO:6). The recombinant gliadin protein of the present invention can also be a dimer or trimer of D2 or its variants, separated by any suitable spacer, such as GGGGS (SEQ ID NO:7). One of skill in the art will appreciate that other spacers are useful in the present invention.

In some embodiments, the recombinant deamidated gliadin is a D2 dimer. In other embodiments, the recombinant gliadin protein is a D2 trimer (SEQ ID NO:2). In some other embodiments, the present invention provides any nucleotide sequence that encodes the polypeptide in SEQ ID NO:1 or SEQ ID NO:2. The recombinant deamidated gliadin proteins of the present invention bind to anti-deamidated gliadin antibodies, and are thus able to identify subjects suffering from gluten related disorders such as celiac disease. One of skill in the art will appreciate that other recombinant deamidated gliadin proteins are useful in the present invention.

The gliadin fusion protein also includes a tag. Any tag known in the art is useful in the gliadin fusion proteins of the present invention. Tags suitable in the antigen of the present invention include, but are not limited to, a Glutathione S-transferase (GST), His-tag, FLAG, Streptag II, HA-tag, Softag 1, Softag 3, c-myc, T7-tag, S-tag, Elastin-like peptides, Chitin-binding domain, thioredoxin, Xylanase 10A, Maltose binding protein and NusA. In some embodiments, the tag is GST or His-tag. One of skill in the art will appreciate that other tags are useful in the present invention.

In another embodiment, the tag is a Glutathione S-transferase (GST) protein. The GST protein (SEQ ID NO:3) serves many functions, including enabling the purification of the recombinant gliadin protein and the presentation of epitopes represented in the recombinant gliadin protein.

When the gliadin fusion protein includes GST and the recombinant deamidated gliadin is the D2 trimer, the gliadin fusion protein is represented by SEQ ID NO:4. In some embodiments, the present invention provides any nucleotide sequence that encodes the polypeptide in SEQ ID NO:4. The gliadin fusion protein of the present invention can be prepared by a variety of methods, including via recombinant methods such as those described.

Immobilization of the gliadin fusion protein on the solid support can be achieved by any method known in the art. The immobilization of the gliadin fusion protein to the solid support can be via covalent or ionic bond formation, hydrogen bonding, Van der Waals forces, as well as via antibody-antigen interactions. One of skill in the art will appreciate that other immobilization methods are useful in the present invention.

In some embodiments, the antigen also includes tissue Transglutaminase (tTG). When tTG is present, the tTG and gliadin fusion protein form a tTG-gliadin fusion protein complex. The tTG and the gliadin fusion protein can be linked in a variety of ways, such as by the formation of covalent bonds, ionic bonds, hydrogen bonding, or by Van der Waals interactions. When the tTG and the gliadin fusion protein are linked covalently, the covalent bonds can be formed by a variety of reactions, such as transamidation. The transamidation can occur under a variety of conditions, such as in the presence of Ca²⁺. The tTG can be linked to either or both of the tag and the recombinant deamidated gliadin of the gliadin fusion protein. The tTG is immobilized to the solid support under the same conditions, and at the same time as immobilization of the gliadin fusion protein. Tissue transglutaminase is known to one of skill in the art and has been described previously, see NCBI RefSeq NP_004604 and NP_945189 (April 13, 2008).

In other embodiments, the tTG and the gliadin fusion protein are covalently linked by a cross-linker. One of skill in the art will appreciate that other methods of cross-linking are available, such as via ionic bonding, hydrogen bonding or via van der Waals forces. One of skill in the art will recognize that any cross-linker is suitable in the instant invention. In some embodiments, the cross-linker is a member selected from the group consisting of a heterobifunctional crosslinker and a homobifunctional crosslinker. In yet other embodiments, the cross-linker is a homobifunctional crosslinker. In still yet other embodiments, the cross-linker is a member selected from the group consisting of bis(sulfosuccinimidyl)suberate (BS3), ethylene glycol bis[succinimidylsuccinate] (EGS), ethylene glycol bis[sulfosuccinimidylsuccinate] (sulfo-EGS), bis[2-(succinimidooxycarbonyloxy)ethyl]sulfone (BSOCOES), dithiobis(succinimidyl)propionate (DSP), 3,3'-dithiobis(sulfosuccinimidylpropionate) (DTSSP), disuccinimidyl suberate (DSS), disuccinimidyl glutarate (DSG), methyl N-succinimidyl adipate (MSA), disuccinimidyl tartarate (DST), 1,5-difluoro-2,4-dinitrobenzene (DFDNB), 1-ethyl-3-[3-dimethylaminopropyl]carbodiimide hydrochloride (EDC or EDAC), sulfosuccinimidyl-4-(N-maleimidomethyl)cyclohexane-1-carboxylate (sulfo-SMCC), N-hydroxysulfosuccinimide (sulfo-NHS), hydroxylamine and Sulfo-LC-SPDP (N-succinimidyl 3-(2-pyridyldithio)-propionate) and sulfosuccinimidyl 6-(3'-[2-pyridyldithio]-propionamido)hexanoate (sulfo-LC-SPDP). In another embodiment, the cross-linker is bis(sulfosuccinimidyl)suberate (BS3).

In a further embodiment, the recombinant deamidated gliadin has 95% identity to SEQ ID NO:2. One of skill in the art will appreciate that other percent identities are possible, such as 60% identity, preferably 65%, 70%, 75%, 80%, 85%, 90%, 95%, 96%, 97%, 98% or 99% identity over a specified region, when compared and aligned for maximum correspondence over a comparison window, or designated region. Such sequences are then said to be "substantially identical." The recombinant deamidated gliadin of the present invention having some percent identity to SEQ ID NO:2 can bind to anti-gliadin antibodies in a sample in order to detect celiac disease. In some other embodiments, the recombinant deamidated gliadin has SEQ ID NO:2.

### B. Solid Support

A solid support material for use in the present invention is characterized by the following properties: (1) insolubility in liquid phases used for screening; (2) capable of mobility in three dimensions independent of all other supports; (3) containing many copies of the gliadin fusion protein or the tTG-gliadin fusion protein complex; (4) compatibility with screening assay conditions; and (5) being inert to the assay conditions. A preferred support also has reactive functional groups, including, but not limited to, hydroxyl, carboxyl, amino, thiol, aldehyde, halogen, nitro, cyano, amido, urea, carbonate, carbamate, isocyanate, sulfone, sulfonate, sulfonamide, sulfoxide, etc., for attaching the gliadin fusion protein and tTG.

As used herein, solid support material is not limited to a specific type of support. Rather a large number of supports are available and are known to one of ordinary skill in the art. Solid phase supports include silica gels, resins, derivatized plastic films, beads such as glass or plastic beads, cotton, alumina gels, polysaccharides such as Sepharose and the like, etc. Other solid supports can be ELISA microtiter plates. A suitable solid phase support can be selected on the basis of desired end use and suitability for various synthetic protocols. For example, in polyamide synthesis, useful solid phase support can be resins such as polystyrene (e.g., PAM-resin obtained from Bachem Inc., Peninsula Laboratories, etc.), POLYHIPE™ resin (obtained from Aminotech, Canada), polyamide resin (obtained from Peninsula Laboratories), polystyrene resin grafted with polyethylene glycol (TentaGel™, Rapp Polymere, Tubingen, Germany), polydimethyl-acrylamide resin (available from Milligen/Biosearch, California), or PEGA beads (obtained from Polymer Laboratories). Preferred solid phase synthesis supports for specific syntheses are described below. In some embodiments, the solid support is a bead. One of skill in the art will recognize that many types of solid supports are useful in the present invention.

### C. Process for Preparing Recombinant Deamidated Gliadin Antigen

In some embodiments, the present invention provides an antigen for detecting celiac disease prepared by the process including contacting a solid support with a gliadin fusion protein having a recombinant deamidated gliadin covalently linked to a tag, to form a modified solid support where the gliadin fusion protein is immobilized on the modified solid support via the tag. Thus, the antigen for detecting celiac disease is prepared.

The tag is as described above. In some embodiments, the tag is GST or a His-tag. In another embodiment, the tag is GST.

When tTG is present, the process can also include forming a covalent bond between the gliadin fusion protein and the tTG prior to the contacting step to form a tTG-gliadin fusion protein complex. The process of forming a covalent bond between the gliadin fusion protein and the tTG can also occur during and/or after the contacting step. The complexing of the gliadin fusion protein and the tTG can occur by any method known in the art. In some embodiments, the complexation occurs by transamidation to form a covalent bond.

In other embodiments, the process further comprises contacting the modified solid support with a cross-linker to cross-link the gliadin fusion protein and the tTG. In some other embodiments, the cross-linker cross-links the GST protein to the tTG. One of skill in the art will appreciate that any cross-linker is useful in the process of the present invention, such as those described above. The cross-linking can occur via hydrogen-bonding, covalent or ionic bond formation.

### 1. General Recombinant Methods

This invention can employ routine techniques in the field of recombinant genetics for the preparation of recombinant deamidated gliadin polypeptides. Basic texts disclosing the general methods of use in this invention include Sambrook & Russell, Molecular Cloning, A Laboratory Manual (3rd Ed, 2001); Kriegler, Gene Transfer and Expression: A Laboratory Manual (1990); and Current Protocols in Molecular- Biology (Ausubel et al., eds., 1994-1999).

A recombinant deamidated gliadin, or a fusion protein, *e.g*., comprising recombinant deamidated gliadin and GST, can be expressed using techniques well known in the art. Eukaryotic and prokaryotic host cells may be used such as animal cells, insect cells, bacteria, fungi, and yeasts. Methods for the use of host cells in expressing isolated nucleic acids are well known to those of skill and may be found, for example, in the general reference, *supra.* Accordingly, this invention also provides for host cells and expression vectors comprising the nucleic acid sequences described herein.

Nucleic acids encoding a recombinant deamidated gliadin, or a fusion protein can be made using standard recombinant or synthetic techniques. Nucleic acids may be RNA, DNA, or hybrids thereof. One of skill can construct a variety of clones containing functionally equivalent nucleic acids, such as nucleic acids that encode the same polypeptide. Cloning methodologies to accomplish these ends, and sequencing methods to verify the sequence of nucleic acids are well known in the art.

In some embodiments, the nucleic acids are synthesized *in vitro.* Deoxynucleotides may be synthesized chemically according to the solid phase phosphoramidite triester method described by Beaucage & Caruthers, Tetrahedron Letts. 22(20):1859-1862 (1981), using an automated synthesizer, *e.g*., as described in Needham-VanDevanter, et al., Nucleic Acids Res. 12:6159-6168 (1984). In other embodiments, the nucleic acids encoding the desired protein may be obtained by an amplification reaction, *e.g.,* PCR.

One of skill will recognize many other ways of generating alterations or variants of a given polypeptide sequence. Most commonly, polypeptide sequences are altered by changing the corresponding nucleic acid sequence and expressing the polypeptide.

One of skill can select a desired nucleic acid or polypeptide of the invention based upon the sequences referred to herein and the knowledge readily available in the art regarding recombinant deamidated gliadin structure and function. The physical characteristics and general properties of these proteins are known to skilled practitioners.

To obtain high level expression of a recombinant deamidated gliadin, recombinant deamidated gliadin-GST fusion protein, an expression vector is constructed that includes such elements as a promoter to direct transcription, a transcription/translation terminator, a ribosome binding site for translational initiation, and the like. Suitable bacterial promoters are well known in the art and described, *e.g.,* in the references providing expression cloning methods and protocols cited hereinabove. Bacterial expression systems for expressing ribonuclease are available in, *e.g., E. coli, Bacillus sp.,* and Salmonella (*see,* also, Palva, et al., Gene 22:229-235 (1983); Mosbach, et al., Nature 302:543-545 (1983). Kits for such expression systems are commercially available. Eukaryotic expression systems for mammalian cells, yeast, and insect cells are well known in the art and are also commercially available.

In addition to the promoter, the expression vector typically contains a transcription unit or expression cassette that contains all the additional elements required for expression of the nucleic acid in host cells. A typical expression cassette thus contains a promoter operably linked to the nucleic acid sequence encoding the recombinant deamidated gliadin, recombinant deamidated gliadin-GST fusion protein, and signals required for efficient polyadenylation of the transcript, ribosome binding sites, and translation termination. Depending on the expression system, the nucleic acid sequence encoding the recombinant deamidated gliadin, recombinant deamidated gliadin-GST fusion protein, may be linked to a cleavable signal peptide sequence to promote secretion of the encoded protein by the transformed cell.

As noted above, the expression cassette should also contain a transcription termination region downstream of the structural gene to provide for efficient termination. The termination region may be obtained from the same gene as the promoter sequence or may be obtained from different genes.

The particular expression vector used to transport the genetic information into the cell is not particularly critical. Any of the conventional vectors used for expression in eukaryotic or prokaryotic cells may be used. Standard bacterial expression vectors include plasmids such as pBR322 based plasmids, pSKF, pET15b, pET23D, pET-22b(+), and fusion expression systems such as GST and LacZ. Epitope tags can also be added to recombinant proteins to provide convenient methods of isolation, *e.g*., 6-his. These vectors comprise, in addition to the expression cassette containing the coding sequence, the T7 promoter, transcription initiator and terminator, the pBR322 ori site, a bla coding sequence and a lac1 operator.

The vectors comprising the nucleic acid sequences encoding the RNAse molecules or the fusion proteins may be expressed in a variety of host cells, including *E. coli,* other bacterial hosts, yeast, and various higher eukaryotic cells such as the COS, CHO and HeLa cells lines and myeloma cell lines. In addition to cells, vectors may be expressed by transgenic animals, preferably sheep, goats and cattle. Typically, in this expression system, the recombinant protein is expressed in the transgenic animal's milk.

The expression vectors or plasmids of the invention can be transferred into the chosen host cell by well-known methods such as calcium chloride transformation for *E. coli* and calcium phosphate treatment, liposomal fusion or electroporation for mammalian cells. Cells transformed by the plasmids can be selected by resistance to antibiotics conferred by genes contained on the plasmids, such as the amp, gpt, neo and hyg genes.

Once expressed, the expressed protein can be purified according to standard procedures of the art, including ammonium sulfate precipitation, column chromatography (including affinity chromatography), gel electrophoresis and the like (*see,* generally, R. Scopes, Protein Purification, Springer--Verlag, N.Y. (1982), Deutscher, Methods in Enzymology Vol. 182: Guide to Protein Purification., Academic Press, Inc. N.Y. (1990); Sambrook and Ausubel, both *supra.*

In some embodiments, the present invention provides an isolated nucleic acid including SEQ ID NO:5, which encodes the recombinant gliadin protein D2 trimer sequence. In other embodiments, the isolated nucleic acid is in an expression vector. In some other embodiments, the expression vector is in a host cell.

### 2. Immobilization on the Solid Support

The gliadin fusion protein of the present invention can be immobilized to any useful solid support material by any useful immobilization method known in the art. The immobilization of the gliadin fusion protein to the solid support can be via covalent or ionic bond formation, hydrogen bonding, Van der Waals forces, as well as via antibody-antigen interactions. One of skill in the art will appreciate that other immobilization methods are useful in the present invention.

Other compounds have been developed that enable immobilization in a manner similar to antibodies. Certain of these "antibody mimics" use non-immunoglobulin protein scaffolds as alternative protein frameworks for the variable regions of antibodies.

For example, Ladner et al. (U.S. Patent No. 5,260,203) describe single polypeptide chain binding molecules with binding specificity similar to that of the aggregated, but molecularly separate, light and heavy chain variable region of antibodies. The single-chain binding molecule contains the antigen binding sites of both the heavy and light variable regions of an antibody connected by a peptide linker and will fold into a structure similar to that of the two peptide antibody. The single-chain binding molecule displays several advantages over conventional antibodies, including, smaller size, greater stability and are more easily modified.

Ku et al. (Proc. Natl. Acad. Sci. U.S.A. 92(14):6552-6556 (1995)) discloses an alternative to antibodies based on cytochrome b₅₆₂. Ku *et al.* (1995) generated a library in which two of the loops of cytochrome b₅₆₂ were randomized and selected for binding against bovine serum albumin. The individual mutants were found to bind selectively with BSA similarly with anti-BSA antibodies.

Lipovsek et al. (U.S. Patent Nos. 6,818,418 and 7,115,396) discloses an antibody mimic featuring a fibronectin or fibronectin-like protein scaffold and at least one variable loop. Known as Adnectins, these fibronectin-based antibody mimics exhibit many of the same characteristics of natural or engineered antibodies, including high affinity and specificity for any targeted ligand. Any technique for evolving new or improved binding proteins may be used with these antibody mimics.

The structure of these fibronectin-based antibody mimics is similar to the structure of the variable region of the IgG heavy chain. Therefore, these mimics display antigen binding properties similar in nature and affinity to those of native antibodies. Further, these fibronectin-based antibody mimics exhibit certain benefits over antibodies and antibody fragments. For example, these antibody mimics do not rely on disulfide bonds for native fold stability, and are, therefore, stable under conditions which would normally break down antibodies. In addition, since the structure of these fibronectin-based antibody mimics is similar to that of the IgG heavy chain, the process for loop randomization and shuffling may be employed in vitro that is similar to the process of affinity maturation of antibodies in vivo.

Beste et al. (Proc. Natl. Acad. Sci. U.S.A. 96(5):1898-1903 (1999)) discloses an antibody mimic based on a lipocalin scaffold (ANTICALIN®). Lipocalins are composed of a β-barrel with four hypervariable loops at the terminus of the protein. Beste (1999), subjected the loops to random mutagenesis and selected for binding with, for example, fluorescein. Three variants exhibited specific binding with fluorescein, with one variant showing binding similar to that of an anti-fluorescein antibody. Further analysis revealed that all of the randomized positions are variable, indicating that ANTICALIN® would be suitable to be used as an alternative to antibodies.

ANTICALINS® are small, single chain peptides, typically between 160 and 180 residues, which provides several advantages over antibodies, including decreased cost of production, increased stability in storage and decreased immunological reaction.

Hamilton et al. (U.S. Patent No. 5,770,380) discloses a synthetic antibody mimic using the rigid, non-peptide organic scaffold of calixarene, attached with multiple variable peptide loops used as binding sites. The peptide loops all project from the same side geometrically from the calixarene, with respect to each other. Because of this geometric confirmation, all of the loops are available for binding, increasing the binding affinity to a ligand. However, in comparison to other antibody mimics, the calixarene-based antibody mimic does not consist exclusively of a peptide, and therefore it is less vulnerable to attack by protease enzymes. Neither does the scaffold consist purely of a peptide, DNA or RNA, meaning this antibody mimic is relatively stable in extreme environmental conditions and has a long life span. Further, since the calixarene-based antibody mimic is relatively small, it is less likely to produce an immunogenic response.

Murali et al. (Cell Mol Biol 49(2):209-216 (2003)) discusses a methodology for reducing antibodies into smaller peptidomimetics, they term "antibody like binding peptidomemetics" (ABiP) which may also be useful as an alternative to antibodies.

In addition to non-immunoglobulin protein frameworks, antibody properties have also been mimicked in compounds comprising RNA molecules and unnatural oligomers (e.g., protease inhibitors, benzodiazepines, purine derivatives and beta-turn mimics). Alternatively, known binding interactions between, for example, streptavidin and biotin, can be used to bind the gliadin fusion protein to the solid support.

Additional methods for linking the gliadin fusion protein to the solid support include the use of homobifunctional and heterobifunctional linkers. Zero-length cross linking reagents induce the direct conjugation of two ligands without the introduction of any extrinsic material. Agents that catalyze the formation of disulfide bonds belong in this category. Another example is reagents that induce the condensation of carboxy and primary amino groups to form an amide bond, such as carbodiimides, ethylchloroformate, Woodward's reagent K1, carbonyldiimidazole, etc. Homobifunctional reagents carry two identical functional groups, whereas heterobifunctional reagents contain two dissimilar functional groups. A vast majority of the heterobifunctional cross-linking agents contains a primary amine-reactive group and a thiol-reactive group. A novel heterobifunctional linker for formyl to thiol coupling was disclosed by Heindel, N. D. et al., Bioconjugate Chem. 2, 427-430 (1991). In a preferred embodiment, the covalent cross-linking agents are selected from reagents capable of forming disulfide (-S-S-), glycol (-CH(OH) -CH(OH)-), azo (-N=N-), sulfone (-S(=O2)-), or ester (-C(=O)-O-) bridges.

Carboxylic acid groups residing on the surface of paramagnetic latex beads, internally dyed with Luminex dyes, can be converted to N-hydroxysuccinimide esters through the action of N-cyclohexyl-N'-(2-morpholinoethyl)carbodiimide metho-p-toluenesulfonate (CMC) and N-hydroxysuccinimide (NHS). After magnetic separation and washing, a mixture of the gliadin fusion protein and tTG is added in a detergent and buffered saline containing 10mM CaCl₂ at pH 7.4. The suspension is incubated for 1 hour with shaking at room temperature. After washing, the beads are blocked to reduce non-specific binding and then stored in particle diluent.

### III. Method for Determining Whether a Subject is Suffering from Celiac Disease

The present invention provides a method for determining whether a subject is suffering from celiac disease. The method includes contacting a sample of bodily fluid from the subject with an antigen having a gliadin fusion protein immobilized on a solid support, as described above. The method also includes detecting any antibody that has become specifically bound to the antigen, thus indicating the presence of celiac disease in the subject.

The sample of the present invention can be any bodily fluid. In some embodiments, the sample can be aqueous humour, bile, blood and blood plasma, breast milk, interstitial fluid, lymph, mucus, pleural fluid, pus, saliva, serum, sweat, tears, urine, cerebrospinal fluid, synovial fluid or intracellular fluid. In some embodiments, the sample can be a blood sample.

The subject of the present invention can be any mammal. In some embodiments, the subject can be primates (*e.g.,* humans), cows, sheep, goats, horses, dogs, cats, rabbits, rats, mice and the like. In other embodiments, the subject is a human.

The presence of the antibody bound to the solid support immobilized gliadin fusion protein or tTG-gliadin fusion protein complex can be detected by any means known in the art. In some embodiments, the detecting step can be performed using an assay such as ELISA, a RIA or an immunofluorescence assay. In other embodiments, the detecting step can be performed using an enzymatic method. Immunoassays which can be used in the detecting step include, for example, competitive and non-competitive assay systems such as Western blots, radioimmunoassays, ELISA (enzyme linked immunosorbent assay), "sandwich" immunoassays, immunoprecipitation assays, precipitin reactions, gel diffusion precipitin reactions, immunodiffusion assays, agglutination assays, complement-fixation assays, immunoradiometric assays, fluorescent immunoassays, protein A immunoassays, and the like. (See, e.g., Harlow and Lane, Using Antibodies, A Laboratory Manual, Cold Spring Harbor Laboratory, New York (1999).)

The antibody specific for the antigen can be any suitable antibody. In some embodiments, the antibody can be IgA, IgD, IgE, IgG or IgM. In other embodiments, the antibody can be IgG or IgA. One of skill in the art will appreciate that other antibodies are useful in the present invention.

### IV. Kits

In some embodiments, the present invention provides a kit including an antigen as described above, a detection reagent, and optionally at least one of buffers, salts, stabilizers and instructions.

Buffers, salts and stabilizers useful in the present invention include those known to one of skill, and can be found in Gennaro, Ed., Remington's Pharmaceutical Sciences, 18th Edition, Mack Publishing Co. (Easton, Pa.) 1990.

### V. Examples

### Example 1: Preparation of the Gliadin Fusion Protein using the D2 Trimer

This example provides a method for preparing the gliadin fusion protein of the present invention using the D2 Trimer.

A DNA sequence encoding the D2 trimer, SEQ ID NO:2, was prepared, digested with a restriction enzyme and inserted into an expression vector containing a DNA fragment encoding GST, at the C-terminal position of GST, for expression of the gliadin fusion protein, SEQ ID NO:4.

### Example 2: Preparation of the Immobilized-Antigen without tTG

This example provides a method for preparing the antigen of the present invention in the absence of tTG that generally involves immobilization of a gliadin fusion protein (GST-D2 trimer) on a solid support.

### Immobilization of Gliadin Fusion Protein

Into a microfuge tube is placed 8 mg of carboxyl modified magnetic beads. To the tube is added 800 µL of 50 mM 2-(N-morpholino)ethanesulfonic acid (MES) pH 6.1 in 70% EtOH (ethanol). Mix and magnetically separate. Pipet off and discard the supernatant. Repeat one more time.

Add 400µL of 120 mM N-hydroxysuccinimide (NHS) in 50mM MES pH 6.1 in 70% EtOH into the tube and mix. Add 400µL of 100mM N-Cyclohexyl-N'-(2-morpholinoethyl)carbodiimide metho-p-toluenesulfonate (CMC) in 50mM MES pH 6.1 in 70% EtOH into the tube and mix. Mix for 30 minutes at room temperature.

Separate the beads from the supernatant and add 800µL of 5mM MES pH 6.1. Mix, magnetically separate, pipette off and discard the supernatant. Repeat one more time.

Suspend the washed particles by adding 200µL of 5mM MES to the tube and mix. Add a mixture of the gliadin fusion protein prepared in Example 1 (GST-D2 trimer) in 600µL of buffered saline containing a detergent. Mix for 60 minutes at room temperature. After the incubation is complete, magnetically separate, pipet off and discard the supernatant.

Add 800µL Post-Coating Wash Buffer (buffered saline containing detergents, preservatives and calcium chloride) to the tube, mix and magnetically separate. Pipet off and discard the supernatant. Repeat 3 more times.

### Bead blocking

Add 800µL of Blocking Buffer (high protein containing buffered saline with detergents, preservatives and blockers) to the tube. Mix for 60 minutes at 2°-8°C. Magnetically separate. Pipet off and discard the supernatant.

Add 800µL of Particle Diluent (buffered saline containing detergents, calcium chloride, preservatives and blockers) to the tube. Mix and then magnetically separate. Pipet off and discard the supernatant. Repeat 3 more times.

Add 800µL of Particle Diluent (100µL/mg particles) into the tube and store at 2°-8°C in this buffer.

### Example 3: Preparation of the Immobilized-Antigen with tTG

This example provides a method for preparing the antigen of the present invention using tTG that involves immobilization of the gliadin fusion protein (GST-D2 trimer) and tTG onto the solid support such that the tTG and gliadin fusion protein become complexed together through transamidation reactions. The tTG and gliadin fusion protein are then cross-linked.

### Immobilization of Gliadin Fusion Protein-tTG Complex

Into a microfuge tube is placed 8 mg of carboxyl modified magnetic beads. To the tube is added 800 µL of 50 mM 2-(N-morpholino)ethanesulfonic acid (MES) pH 6.1 in 70% EtOH (ethanol). Mix and magnetically separate. Pipet off and discard the supernatant. Repeat one more time.

Add 400µL of 120 mM N-hydroxysuccinimide (NHS) in 50mM MES pH 6.1 in 70% EtOH into the tube and mix. Add 400µL of 100mM N-Cyclohexyl-N'-(2-morpholinoethyl)carbodiimide metho-p-toluenesulfonate (CMC) in 50mM MES pH 6.1 in 70% EtOH into the tube and mix. Mix for 30 minutes at room temperature.

Separate the beads from the supernatant and add 800µL of 5mM MES pH 6.1. Mix, magnetically separate, pipette off and discard the supernatant. Repeat one more time.

Suspend the washed particles by adding 200µL of 5mM MES to the tube and mix. Add a mixture of the gliadin fusion protein prepared above (GST-D2 trimer) and tTG in 600µL of buffered saline containing a detergent and calcium chloride. Mix for 60 minutes at room temperature. After the incubation is complete, magnetically separate, pipet off and discard the supernatant.

Add 800µL Post-Coating Wash Buffer (buffered saline containing detergents, preservatives and calcium chloride) to the tube, mix and magnetically separate. Pipet off and discard the supernatant. Repeat 3 more times.

Add 800µL buffered saline containing calcium chloride pH7.4 to the tube, mix and magnetically separate. Pipet off and discard the supernatant. Repeat 3 more times.

Add 800µL of 32 mM suberic acid bis sulfo(n-hydroxysuccinimide (BS3) in buffered saline containing calcium chloride pH 7.4 into the tube and mix. Mix for 30 minutes at room temperature. After the incubation is complete, magnetically separate, pipet off and discard the supernatant.

Add 800µL Post-Coating Wash Buffer (buffered saline containing detergents, preservatives and calcium chloride) to the tube, mix and magnetically separate. Pipet off and discard the supernatant. Repeat 3 more times.

### Bead blocking

Add 800µL of Blocking Buffer (high protein containing buffered saline with detergents, calcium chloride, preservatives and blockers) to the tube. Mix for 60 minutes at 2°-8°C. Magnetically separate. Pipet off and discard the supernatant.

Add 800µL of Particle Diluent (buffered saline containing detergents, calcium chloride, preservatives and blockers) to the tube. Mix and then magnetically separate. Pipet off and discard the supernatant. Repeat 3 more times.

Add 800µL of Particle Diluent (100µL/mg particles) into the tube and store at 2°-8°C in this buffer.

### Example 4: Detection of Celiac Disease using the Antigen

This example provides a method for detection of celiac disease using the recombinant deamidated gliadin antigen of the present invention.

A summary of the Gastrointestinal IgA and IgG method follows.
- The instrument (BioPlex 2200™ manufactured by Bio-Rad Laboratories) aspirates 5 µL of sample from the sample tube and dispenses it into a reaction vessel (RV) chased by 45 µL of Wash Buffer (phosphate buffered saline containing detergent and preservatives).
- To the RV are added 100 µL of Sample Diluent (buffered saline containing detergents, preservatives and blockers) and 150 µL of Wash Buffer.
- The RV is incubated for 130 seconds (2.2 minutes) at 37°C.
- To the RV is added 100 µL of Particle Reagent (a solution of recombinant deamidated gliadin antigen coated beads and Gliadin Fusion Protein-tTG Complex antigen coated beads prepared in Examples 2 and 3, respectively, and particle diluent). The final sample dilution is 1/80.
- The mixture is incubated for 1180 seconds (19.7 minutes) at 37°C with intermittent mixing.
- The beads are washed 3-times with 600 then 300 then 600 µL of Wash Buffer.
- 50 µL of Conjugate Reagent is added to RV (a mixture of anti-human IgA-phycoerythrin in conjugate diluent (buffered saline containing detergents, preservatives and blockers)).
- The mixture is incubated for 600 seconds (10 minutes) at 37°C with intermittent mixing.
- The beads are washed 3-times with 600 then 300 then 600 µL of Wash Buffer.
- 50 µL of Wash Buffer is added to the RV.
- The bead suspension is aspirated into the Luminex Detector Module (LDM) and the median fluorescence of particles in each of the specified bead regions is measured.

### Example 5: Sensitivity in Celiac Disease Testing

The study was comprised of 122 Celiac samples (consuming gluten in the daily diet) along with 30 other IBD samples and 194 normal healthy samples.

| **Antibody** | **Analyte** | **Clinical Agreement (%)** | | |
|---|---|---|---|---|
| | | **Positive Agreement** | **Negative Agreement** | **Total Agreement** |
| IgA | tTG | 74 | 98 | 89 |
| | D2 | 70 | 98 | 88 |
| | D2-tTG | 77 | 97 | 90 |
| IgG | tTG | 22 | 100 | 73 |
| | D2 | 69 | 98 | 88 |
| | D2-tTG | 56 | 99 | 84 |

| | | | | |
|---|---|---|---|---|
| tTG = bead coated with tissue Transglutaminase; D2 = bead coated with D2 trimer; D2-tTG = bead coated with complex of D2 and tTG. | | | | |

| **Antibody** | **Analyte** | **Number of Celiac Positives** |
|---|---|---|
| IgA | tTG | 90 |
| | D2 | 86 |
| | D2-tTG | 94 |
| IgG | tTG | 27 |
| | D2 | 83 |
| | D2-tTG | 68 |

| | | |
|---|---|---|
| tTG = bead coated with tissue Transglutaminase; D2 = bead coated with D2 trimer; D2-tTG = bead coated with complex of D2 and tTG. | | |

A second study comprised 125 Celiac samples (consuming gluten in the daily diet) and 198 normal healthy samples.

| **Antibody** | **Analyte** | **Clinical Agreement (%)** | | |
|---|---|---|---|---|
| | | **Positive Agreement** | **Negative Agreement** | **Total Agreement** |
| IgA | Gliadin | 38 | 98 | 75 |
| | tTG | 76 | 98 | 90 |
| | Gliadin Fusion Protein | 73 | 98 | 88 |
| IgG | Gliadin | 18 | 98 | 67 |
| | tTG | 38 | 98 | 75 |
| | Gliadin Fusion Protein | 67 | 98 | 86 |

| | | | | |
|---|---|---|---|---|
| Gliadin = bead coated with whole natural gliadin; tTG = bead coated with tTG (tissue transglutaminase); and Gliadin Fusion Protein = bead coated with recombinant deamidated gliadin fused to GST protein | | | | |

### Example 6: Comparative Data of D2 trimer vs. D2 monomer

Following the procedure of Example 2, a first antigen comprising the D2 trimer was prepared along with a second antigen comprising the D2 peptide monomer. The two antigens were tested and the D2 trimer antigen achieved a higher maximum signal than the D2 peptide monomer. See Figure 1.

| **Coating** | **Coating Conc (pmol/mg)** | | **Cutoff Signal (RFI)** | |
|---|---|---|---|---|
| **Conc. (µg/mg)** | **D2 Peptide** | **D2-Trimer** | **D2 Peptide** | **D2-Trimer** |
| 0.01 | 4 | -- | 152 | -- |
| 0.03 | 13 | -- | 235 | -- |
| 0.1 | 42 | 3 | 235 | 75 |
| 0.3 | 126 | 9 | 254 | 127 |
| 1 | -- | 30 | -- | 204 |
| 3 | -- | 90 | -- | 316 |
| 10 | -- | 301 | -- | 421 |
| 30 | -- | 904 | -- | 492 |
| 100 | -- | 3012 | -- | 644 |

| | | | | |
|---|---|---|---|---|
| RFI = relative fluorescence intensity | | | | |

The D2 trimer antigen was also found to have better clinical sensitivity than the D2 peptide antigen.

| **Antibody** | **Analyte** | **Positive Agreement (%)** | **Negative Agreement (%)** |
|---|---|---|---|
| IgA | D2 Peptide | 67 | 98 |
| IgA | D2 Trimer | 73 | 98 |
| IgG | D2 Peptide | 66 | 98 |
| IgG | D2 Trimer | 67 | 98 |

Although the foregoing invention has been described in some detail by way of illustration and example for purposes of clarity of understanding, one of skill in the art will appreciate that certain changes and modifications may be practiced within the scope of the appended claims.
**SEQ ID NO:1**
   QPEQPQQSFPEQERPF
**SEQ ID NO:2**
   QPEQPQQSFPEQERPFGGGGSQPEQPQQSFPEQERPFGGGGSQPEQPQQSFPEQERPF
**SEQ ID NO:3** 
MSPILGYWKIKGLVQPTRLLLEYLEEKYEEHLYERDEGDKWRNKKFELGLEFPNLPY
YIDGDVKLTQSMAIIRYIADKHNMLGGCPKERAEISMLEGAVLDIRYGVSRIAYSKDF
ETLKVDFLSKLPEMLKMFEDRLCHKTYLNGDHVTHPDFMLYDALDVVLYMDPMCL
DAFPKLVCFKKRIEAIPQIDKYLKSSKYIAWPLQGWQATFGGGDHPPKSDLVPR
**SEQ ID NO:4** 
MSPILGYWKIKGLVQPTRLLLEYLEEKYEEHLYERDEGDKWRNKKFELGLEFPNLPY
YIDGDVKLTQSMAIIRYIADKHNMLGGCPKERAEISMLEGAVLDIRYGVSRIAYSKDF
ETLKVDFLSKLPEMLKMFEDRLCHKTYLNGDHVTHPDFMLYDALDVVLYMDPMCL
DAFPKLVCFKKRIEAIPQIDKYLKSSKYIAWPLQGWQATFGGGDHPPKSDLVPRQPE
QPQQSFPEQERPFGGGGSQPEQPQQSFPEQERPFGGGGSQPEQPQQSFPEQERPF
**SEQ ID NO:5**

CAGCCC GAACAACCGC AACAATCATT CCCCGAGCAA GAAAGGCCGT
TCGGTGGCGG TGGCTCGCAG CCCGAACAAC CGCAACAATC ATTCCCCGAG
CAAGAAAGGC CGTTCGGTGG CGGTGGCTCG CAGCCCGAAC AACCGCAACA
ATCATTCCCC GAGCAAGAAA GGCCGTTC

Described herein are:
An antigen for detecting celiac disease comprising a recombinant deamidated gliadin covalently linked to a tag to form a gliadin fusion protein, wherein the tag is immobilized on a solid support.

The antigen as defined above, wherein the tag is selected from the group consisting of a Glutathione S-transferase (GST) and a His-tag.

The antigen,wherein the tag is GST.

The antigen as defined above, wherein the antigen further comprises tissue Transglutaminase (tTG) to form a tTG-gliadin fusion protein complex.

The antigen as defined above, wherein the tTG and the gliadin fusion protein are covalently linked by a cross-linker.

The antigen as defined above, wherein the cross-linker is a member selected from the group consisting of a heterobifunctional crosslinker and a homobifunctional crosslinker.

The antigen as defined above, wherein the cross-linker is a homo bifunctional crosslinker. The antigen as defined above, wherein the cross-linker is a member selected from the group consisting of bis(sulfosuccinimidyl)suberate (BS3), ethylene glycol bis[succinimidylsuccinate] (EGS), ethylene glycol bis[sulfosuccinimidylsuccinate] (sulfo-EGS), bis[2-(succinimidooxycarbonyloxy)ethyl]sulfone (BSOCOES), dithiobis(succinimidyl)propionate (DSP), 3,3'-dithiobis(sulfosuccinimidylpropionate) (DTSSP), disuccinimidyl suberate (DSS), disuccinimidyl glutarate (DSG), methyl N-succinimidyl adipate (MSA), disuccinimidyl tartarate (DST), 1,5-difluoro-2,4-dinitrobenzene (DFDNB), 1-ethyl-3-[3-dimethylaminopropyl]carbodiimide hydrochloride (EDC or EDAC), sulfosuccinimidyl-4-(N-maleimidomethyl)cyclohexane-1-carboxylate (sulfo-SMCC), N-hydroxysulfosuccinimide (sulfo-NHS), hydroxylamine and Sulfo-LC-SPDP (N-succinimidyl 3-(2-pyridyldithio)-propionate) and sulfosuccinimidyl 6-(3'-[2-pyridyldithio]-propionamido)hexanoate (sulfo-LC-SPDP).

The antigen as defined above, wherein the cross-linker is bis(sulfosuccinimidyl)suberate (BS3).

The antigen as defined above, wherein the recombinant deamidated gliadin has 95% identity to SEQ ID NO:2.

The antigen as defined above, wherein the recombinant deamidated gliadin has SEQ ID NO:2.

An antigen for detecting celiac disease prepared by the process comprising:
(a) contacting a solid support with a gliadin fusion protein, wherein the gliadin fusion protein comprises a recombinant deamidated gliadin covalently linked to a tag, such that the gliadin fusion protein is immobilized on the solid support via the tag, thereby preparing the antigen for detecting celiac disease.

The antigen as defined above, wherein the tag is selected from the group consisting of a Glutathione S-transferase (GST) and a His-tag.

The antigen as defined above, wherein the process further comprises contacting the gliadin fusion protein with a tissue Transglutaminase (tTG) prior to contacting step (a) to form at least one covalent bond between the gliadin fusion protein and the tTG.

The antigen as defined above, wherein the process further comprises
(b) contacting the antigen with a cross-linker to cross-link the gliadin fusion protein to the tTG.

A method for determining whether a subject is suffering from celiac disease, the method comprising:
(a) contacting a sample of bodily fluid from the subject with the antigen as defined above; and
(b) detecting any antibody that has become specifically bound to the antigen, as an indication of the presence of celiac disease in the subject.

The method as defined above, wherein the sample is a blood sample.

The method as defined above, wherein the detecting step is performed using an assay selected from the group consisting of ELISA, a RIA and an immunofluorescence assay.

The method as defined above, wherein the antibody specific for the antigen is selected from the group consisting of IgG and IgA.

A kit comprising an antigen as defined above; a detection reagent; and optionally at least one member selected from the group consisting of buffers, salts, stabilizers and instructions.

An isolated nucleic acid comprising SEQ ID NO:5.

The isolated nucleic acid as defined above, in an expression vector.

The isolated nucleic acid as defined above, wherein the expression vector is in a host cell.

## Claims

1. An antigen for detecting celiac disease, comprising a recombinant or synthetic deamidated gliadin protein comprising a dimer or trimer of SEQ ID NO:1.

2. The antigen of claim 1, wherein the recombinant or synthetic deamidated gliadin is immobilized on a solid support.

3. The antigen of claim 1, wherein the recombinant or synthetic deamidated gliadin is covalently linked to a tag to form a gliadin fusion protein, wherein the tag is immobilized on a solid support.

4. The antigen of claim 3, wherein the tag is selected from a Glutathione S-transferase (GST) and a His-tag, preferably
(i) the tag is GST; or
(ii) the antigen further comprises tissue Transglutaminase (tTG) to form a tTG-gliadin fusion protein complex, most preferably the tTG and the gliadin fusion protein are covalently linked by a cross-linker.

5. The antigen of claim 4, wherein the cross-linker is a member selected from a heterobifunctional crosslinker and a homobifunctional crosslinker, preferably the cross-linker is a homobifunctional crosslinker, more preferably the cross-linker is selected from bis(sulfosuccinimidyl)suberate (BS3), ethylene glycol bis[succinimidylsuccinate] (EGS), ethylene glycol bis[sulfosuccinimidylsuccinate] (sulfo-EGS), bis[2-(succinimidooxycarbonyloxy)ethyl]sulfone (BSOCOES), dithiobis(succinimidyl)propionate (DSP), 3,3'-dithiobis(sulfosuccinimidylpropionate) (DTSSP), disuccinimidyl suberate (DSS), disuccinimidyl glutarate (DSG), methyl N-succinimidyl adipate (MSA), disuccinimidyl tartarate (DST), 1,5-difluoro-2,4-dinitrobenzene (DFDNB), 1-ethyl-3-[3- dimethylaminopropyl]carbodiimide hydrochloride (EDC or EDAC), sulfosuccinimidyl-4-(N- maleimidomethyl)cyclohexane-1-carboxylate (sulfo-SMCC), N-hydroxysulfosuccinimide (sulfo-NHS), hydroxylamine and Sulfo-LC-SPDP (N-succinimidyl 3-(2-pyridyldithio)-propionate) and sulfosuccinimidyl 6-(3'-[2-pyridyldithio]-propionamido)hexanoate (sulfo-LC-SPDP), most preferably the cross-linker is bis(sulfosuccinimidyl)suberate (BS3).

6. The antigen of any one of claims 1 to 5, wherein the recombinant or synthetic deamidated gliadin has 95% identity to SEQ ID NO:2, preferably the recombinant or synthetic deamidated gliadin has SEQ ID NO:2.

7. A process for preparing the antigen for detecting celiac disease according to claim 3, said process comprising:
(a) contacting a solid support with a gliadin fusion protein, wherein the gliadin fusion protein comprises a recombinant or synthetic deamidated gliadin covalently linked to a tag, such that the gliadin fusion protein is immobilized on the solid support via the tag, thereby preparing the antigen for detecting celiac disease.

8. The process of claim 7, which further comprises contacting the gliadin fusion protein with a tissue Transglutaminase (tTG) prior to contacting step (a) to form at least one covalent bond between the gliadin fusion protein and the tTG, preferably the process further comprises: (b) contacting the antigen with a cross-linker to cross-link the gliadin fusion protein to the tTG.

9. A method for determining whether a subject is suffering from celiac disease, the method comprising:
(a) contacting a sample of bodily fluid from the subject with the antigen according to any one of claims 1 to 6; and
(b) detecting any antibody that has become specifically bound to the antigen, as an indication of the presence of celiac disease in the subject.

10. The method of claim 8, wherein
(i) the sample is a blood sample;
(ii) the detecting step is performed using an assay selected from the group cohsisting of ELISA, a RIA and an immunofluorescence assay; and/or
(iii) the antibody specific for the antigen is selected from the group consisting of IgG and IgA.

11. A kit comprising an antigen according to any one of claims 1 to 6; a detection reagent; and optionally at least one member selected from the group consisting of buffers, salts, stabilizers and instructions.

12. An isolated nucleic acid encoding the antigen according to any one of claims 1 to 6 or comprising SEQ ID NO:5.

13. An expression vector comprising the isolated nucleic acid of claim 12.

14. A host cell comprising the expression vector of claim 13.

15. Use of the recombinant or synthetic deamidated gliadin according to any one, of claims 1 to 6 for determining whether a subject is suffering from a celiac disease.

## Patentansprüche

1. Antigen zum Nachweis von Zöliakie, umfassend ein rekombinantes oder synthetisches deamidiertes Gliadinprotein, das ein Dimer oder Trimer von SEQ ID Nr. 1 umfasst.

2. Antigen gemäß Anspruch 1, wobei das rekombinante oder synthetische deamidierte Gliadin auf einem festen Träger immobilisiert ist.

3. Antigen gemäß Anspruch 1, wobei das rekombinante oder synthetische deamidierte Gliadin unter Bildung eines Gliadin-Fusionsproteins kovalent an ein Tag gebunden ist, wobei das Tag auf einem festen Träger immobilisiert ist.

4. Antigen gemäß Anspruch 3, wobei das Tag aus einer Glutathion-S-Transferase (GST) und einem His-Tag ausgewählt ist, wobei vorzugsweise
(i) es sich bei dem Tag um GST handelt; oder
(ii) das Antigen weiterhin Gewebe-Transglutaminase (tTG) umfasst, so dass ein tTG-Gliadin-Fusionsprotein-Komplex entsteht, wobei am meisten bevorzugt die tTG und das Gliadin-Fusionsprotein durch einen Vernetzer kovalent vernetzt sind.

5. Antigen gemäß Anspruch 4, wobei der Vernetzer ein Vertreter ist, der aus einem heterobifunktionellen Vernetzer und einem homobifunktionellen Vernetzer ausgewählt ist, wobei der Vernetzer vorzugsweise ein homobifunktioneller Vernetzer ist, wobei der Vernetzer besonders bevorzugt aus Bis(sulfosuccinimidyl)suberat (BS3), Ethylenglycolbis[succinimidylsuccinat] (EGS), Ethylenglycolbis[sulfosuccinimidylsuccinat] (Sulfo-EGS), Bis[2-(succinimidooxycarbonyloxy)ethyl]sulfon (BSOCOES), Dithiobis-(succinimidyl)propionat (DSP), 3,3'-Dithiobis(sulfosuccinimidylpropionat) (DTSSP), Disuccinimidylsuberat (DSS), Disuccinimidylglutarat (DSG), Methyl-N-succinimidyladipat (MSA), Disuccinimidyltartrat (DST), 1,5-Difluor-2,4-dinitrobenzol (DFDNB), 1-Ethyl-3-[3-dimethylaminopropyl]-carbodiimid-Hydrochlorid (EDC oder EDAC), Sulfosuccinimidyl-4-(N-maleimidomethyl)cyclohexan-1-carboxylat (Sulfo-SMCC), N-Hydroxysulfosuccinimid (Sulfo-NHS), Hydroxylamin und Sulfo-LC-SPDP (N-Succinimidyl-3-(2-pyridyldithio)propionat) und Sulfosuccinimidyl-6-(3'-[2-pyridyldithio]propionamido)hexanoat (Sulfo-LC-SPDP) ausgewählt ist, wobei es sich bei dem Vernetzer am meisten bevorzugt um Bis(sulfosuccinimidyl)suberat (BS3) handelt.

6. Antigen gemäß einem der Ansprüche 1 bis 5, wobei das rekombinante oder synthetische deamidierte Gliadin 95% Identität mit SEQ ID Nr. 2 aufweist, wobei das rekombinante oder synthetische deamidierte Gliadin vorzugsweise die Sequenz SEQ ID Nr. 2 aufweist.

7. Verfahren zum Herstellen des Antigens zum Nachweisen von Zöliakie gemäß Anspruch 3, wobei das Verfahren Folgendes umfasst:
(a) In-Kontakt-Bringen eines festen Trägers mit einem Gliadin-Fusionsprotein, wobei das Gliadin-Fusionsprotein ein rekombinantes oder synthetisches deamidiertes Gliadin umfasst, das kovalent an ein Tag gebunden ist, so dass das Gliadin-Fusionsprotein über das Tag auf dem festen Träger immobilisiert ist, wodurch das Antigen zum Nachweisen von Zöliakie hergestellt wird.

8. Verfahren gemäß Anspruch 7, das vor dem Schritt des In-Kontakt-Bringens (a) weiterhin das In-Kontakt-Bringen des Gliadin-Fusionsproteins mit einer Gewebe-Transglutaminase (tTG) unter Bildung wenigstens einer kovalenten Bindung zwischen dem Gliadin-Fusionsprotein und der tTG umfasst, wobei das Verfahren vorzugsweise weiterhin (b) das In-Kontakt-Bringen des Antigens mit einem Vernetzer unter Vernetzung des Gliadin-Fusionsproteins an die tTG umfasst.

9. Verfahren zum Bestimmen, ob ein Patient an Zöliakie leidet, wobei das Verfahren Folgendes umfasst:
(a) In-Kontakt-Bringen einer Körperflüssigkeitsprobe von dem Patienten mit dem Antigen gemäß einem der Ansprüche 1 bis 6; und
(b) Nachweisen irgendeines Antikörpers, der spezifisch an das Antigen gebunden hat, als Hinweis auf die Anwesenheit von Zöliakie bei dem Patienten.

10. Verfahren gemäß Anspruch 8, wobei
(i) die Probe eine Blutprobe ist;
(ii) der Nachweisschritt unter Verwendung eines Assays durchgeführt wird, der aus der Gruppe ausgewählt ist, die aus einem ELISA, RIA und einem Immunfluoreszenzassay besteht; und/oder
(iii) der für das Antigen spezifische Antikörper aus der Gruppe ausgewählt ist, die aus IgG und IgA besteht.

11. Kit, umfassend ein Antigen gemäß einem der Ansprüche 1 bis 6, ein Nachweisreagens und gegebenenfalls wenigstens einen Vertreter, der aus der Gruppe ausgewählt ist, die aus Puffern, Salzen, Stabilisatoren und Anweisungen besteht.

12. Isolierte Nucleinsäure, die das Antigen gemäß einem der Ansprüche 1 bis 6 codiert oder SEQ ID Nr. 5 umfasst.

13. Expressionsvektor, der die isolierte Nucleinsäure gemäß Anspruch 12 umfasst.

14. Wirtszelle, die den Expressionsvektor gemäß Anspruch 13 umfasst.

15. Verwendung des rekombinanten oder synthetischen deamidierten Gliadins gemäß einem der Ansprüche 1 bis 6 zum Bestimmen, ob ein Patient an Zöliakie leidet.

## Revendications

1. Antigène pour la détection de la maladie coeliaque, comprenant une protéine de type gliadine désamidée, recombinante ou synthétique, comportant un dimère ou un trimère de SEQ ID N0 1.

2. Antigène selon la revendication 1, dans lequel la gliadine désamidée, recombinante ou synthétique, est immobilisée sur un support solide.

3. Antigène selon la revendication 1, dans lequel la gliadine désamidée, recombinante ou synthétique, est liée de manière covalente à un marqueur de façon à former une protéine de fusion de type gliadine, ledit marqueur étant immobilisé sur un support solide.

4. Antigène selon la revendication 3, dans lequel le marqueur est sélectionné parmi un marqueur Glutathion-S-Transférase (GST) et un marqueur His, de préférence
(i) le marqueur est une GST ; ou
(ii) l'antigène comprend en outre une transglutaminase tissulaire (tGT) de façon à former un complexe protéine de fusion de type gliadine-tGT, de façon préférée entre toutes la tGT et la protéine de fusion de type gliadine sont liées de manière covalente par un agent réticulant.

5. Antigène selon la revendication 4, dans lequel l'agent réticulant est un élément sélectionné parmi un agent réticulant hétérobifonctionnel et un agent réticulant homobifonctionnel, de préférence l'agent réticulant est homobifonctionnel, plus préférablement l'agent réticulant est sélectionné parmi : du bis(sulfosuccinimidyl)subérate (BS3), du bis[succinimidylsuccinate] d'éthylène glycol (EGS), du bis[sulfosuccinimidylsuccinate] d'éthylène glycol (sulfo-EGS), de la bis[2-(succinimido-oxycarbonyloxy)éthyl]sulfone (BSOCOES), du dithiobis(succinimidyl)propionate (DSP), du 3,3'-dithiobissulfosuccinimidylproprionate) (DTSSP), du subérate de disuccinimidyle (DSS), du glutarate de disuccinimidyle (DSG), de l'adipate de méthyl-N-succinimidyle (MSA), du tartrate de disuccinimidyle (DST), du 1,5-difluoro-2,4-dinitrobenzène (DFDNB), du chlorhydrate de 1-éthyl-3-[3-diméthylaminopropyl]carbodiimide (EDC ou EDAC), du sulfosuccinimidyl-4-(N-maléimidométhyl)cyclohexane-1-carboxylate (sulfo-SMCC), du N-hydroxysulfosuccinimide (sulfo-NHS), de l'hydroxylamine et du sulfo-LC-SPDP (N-succinimidyl-3-(2-pyridyldithio)-propionate et du sulfosuccinimidyl-6-(3'-[2-pyridyldithio]-propionamido)hexanoate (sulfo-LC-SPDP), l'agent réticulant étant de façon préférée entre toutes le bis(sulfosuccinimidyl)subérate (BS3).

6. Antigène selon l'une quelconque des revendications 1 à 5, dans lequel la gliadine désamidée, recombinante ou synthétique, présente une identité de séquence de 95 % avec la SEQ ID N0. 2, de préférence la gliadine désamidée, recombinante ou synthétique présente la SEQ ID N0 2.

7. Procédé de préparation de l'antigène pour la détection de la maladie coeliaque selon la revendication 3, ledit procédé comprenant les étapes suivantes :
(a) mettre en contact un support solide avec une protéine de fusion de type gliadine, ladite protéine de fusion de type gliadine comprenant une gliadine désamidée, recombinante ou synthétique, liée de manière covalente à un marqueur, de sorte que la protéine de fusion de type gliadine est immobilisée sur le support solide via le marqueur, moyennant quoi l'antigène pour la détection de la maladie coeliaque est préparé.

8. Procédé selon la revendication 7, comprenant en outre l'étape la mise en contact de la protéine de fusion de type gliadine avec une transglutaminase tissulaire (tGT) avant l'étape de contact (a), afin de former au moins une liaison covalente entre la protéine de fusion de type gliadine et la tGT, de préférence ledit procédé comprend en outre une étape (b) la mise en contact de l'antigène avec un agent réticulant pour réticuler la protéine de fusion de type gliadine avec la tGT.

9. Procédé d'analyse permettant d'identifier chez un sujet la présence ou non de la maladie coeliaque, ledit procédé comprenant:
(a) mettre en contact un échantillon de fluide corporel prélevé chez un sujet avec l'antigène selon l'une quelconque des revendications 1 à 6 ; et
(b) détecter tout anticorps s'étant spécifiquement lié à l'antigène, en tant qu'indication de la présence de la maladie coeliaque chez le sujet.

10. Procédé selon la revendication 8, dans lequel
(i) l'échantillon est un échantillon sanguin ;
(ii) l'étape de détection est réalisée à l'aide d'un dosage sélectionné dans le groupe constitué des méthodes immuno-enzymatique (ELISA), radio-immunoessai et immunofluorescence ; et/ou
(iii) l'anticorps spécifique de l'antigène est sélectionné dans le groupe constitué des immunoglobulines IgG et IgA.

11. Trousse comprenant un antigène selon l'une quelconque des revendications 1 à 6, un réactif de détection, et, en option, au moins un élément sélectionné dans le groupe constitué de tampons, de sels, d'agents stabilisants et d'instructions.

12. Acide nucléique isolé codant pour un antigène selon l'une quelconque des revendications 1 à 6 ou comprenant la SEQ ID N0 5.

13. Vecteur d'expression comprenant l'acide nucléique isolé selon la revendication 12.

14. Cellule hôte comprenant le vecteur d'expression selon la revendication 13.

15. Utilisation de la gliadine désamidée, recombinante ou synthétique selon l'une quelconque des revendications 1 à 6 pour identifier chez un sujet la présence ou non de la maladie coeliaque.
